# EUROPEAN PATENT APPLICATION

(11) **EP 1 121 937 A1**
(43) Date of publication of application: **08.08.2001**
(21) Application number: 99943341.0
(22) Date of filing: 14.09.1999
(51) Int. Cl.: A61K 39/39

(54) **VACCINE PREPARATIONS CONTAINING SAPONINS**

(30) Priority: 14.09.1998 JP 25978398
(71) Applicant: THE KITASATO INSTITUTE, Tokyo 108-8642 (JP)
(72) Inventor: YAMADA, Haruki, Oriental Medicine Res. Center, Minato-ku, Tokyo 108-8642 (JP); KIYOHARA, Hiroaki, Oriental Medicine Res. Center, Minato-ku, Tokyo 108-8642 (JP); NAGAI, Takayuki, Oriental Medicine Res. Center, Minato-ku, Tokyo 108-8642 (JP); YABE, Takeshi, Oriental Medicine Res. Center, Minato-ku, Tokyo 108-8642 (JP); AIZAWA, Chikara, Res. Center Biologicals, Kitamoto-shi, Saitama 364-0026 (JP); SUZUKI, Yuuziro, Res. Center Biologicals, Kitamoto-shi, Saitama 364-0026 (JP); SUSA, Eizaburo, Res. Center Biologicals, Kitamoto-shi, Saitama 364-0026 (JP); KATO, Toshio, Res. Center Biologicals, Kitamoto-shi, Saitama 364-0026 (JP); NAGAMINE, Takashi, Res. Center Biologicals, Kitamoto-shi, Saitama 364-0026 (JP)
(74) Representative: Grünecker, Kinkeldey, Stockmair & Schwanhäusser Anwaltssozietät
(86) International application number: JP9905019
(87) International publication number: WO0015257

(57) **Abstract**

The present invention provides an adjuvant comprising, as an active ingredient, a saponin compound having a presenegenin skeleton substituted with a substituted or unsubstituted sugar residue at position 28, and provides the vaccine preparation comprising the adjuvant. For example, sufficient immunological stimulation can be attained even in the intranasal inoculation by utilizing the saponin having the structure shown below.

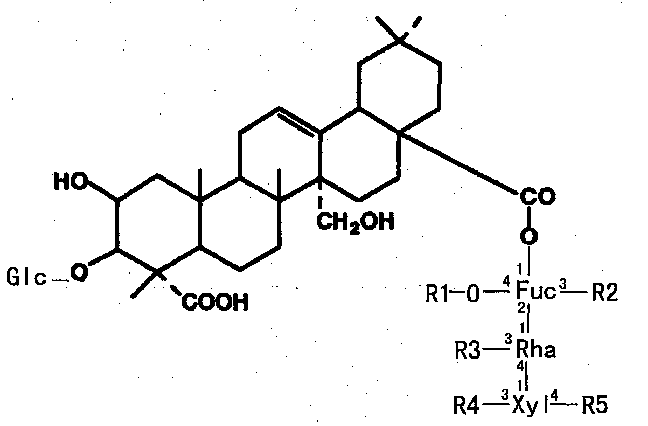

## Description

### Technical Field

The present invention relates to an adjuvant of which active ingredient is saponin and relates to vaccine preparations containing the adjuvant, which is useful to prevent or treat diseases of human and other animals.

### Background Art

Vaccine has been used to prevent various diseases, and has been produced tremendous and excellent results for preventing specific diseases such as smallpox. Nonetheless, vaccine also has side effects and there are many cases in which vaccines are less effective. Thus vaccines still have much room for further improvements. Currently, many types of vaccines used for human or other animals are prepared by using pathogenic organisms or parts thereof as antigenic materials for vaccine production. Thus, there is no denying the possibility that vaccines are contaminated with constituents of pathogenic organisms or ingredients of growth medium for pathogenic organisms. These contaminants can be a cause of adverse side effects in vaccination. In addition, antigenic sites themselves associated with immunization can induce side effects when inoculated in large quantity.

In order to avoid such side effects as much as possible and manufacture excellent vaccines in safety, some attempts have been made, which include the reduction of inoculum dose of vaccine, high-purity preparation of antigen for vaccine, alteration of vaccination route, and others. However, these revisions have a general problem that the immunological activity of vaccine tends to be reduced associated with the revisions. Previously, adjuvant has been used to prevent the decline of immunological activity. However, regarding publicly known adjuvants, there remains some problems to be improved in effectiveness, safety, etc.

In general, most vaccines are inoculated by injection. This results in elevation of antibody titer in the blood. When the titer is kept at a high level, it is possible to prevent a disease caused by pathogenic microorganism. On the other hand, an aerial infectious microorganism like influenza virus infects via mucous membranes of the respiratory tract. To prevent such diseases at early stages of infection, vaccines capable of significantly enhancing local immunity on the mucous membrane rather than in the blood are preferred. In this context, it is also preferable to have an adjuvant capable of contributing to the enhancement of local immunity. In other words, to develop an excellent adjuvant that is effective and safe and that helps the enhancement of required immunity is an important challenge for the development of vaccine.

Instead of injection, oral or intranasal inoculation is noteworthy as a vaccination route. The injection must be performed by medical technicians. Therefore, the vaccination by injection is problematic, for example, when it is necessary to vaccinate many people under a condition with no or only poor medical facilities. On the other hand, oral inoculation or intranasal inoculation can be performed without direct practices by medically skilled staffs as long as vaccine preparations are available. However, in general, sufficient immunological stimulation is difficult to attain with these types of vaccination routes and, therefore, certain adjuvants are needed.

Previously, aluminum compounds (aluminum sulfate, aluminum hydroxide, aluminum phosphate, etc.) have widely been used as adjuvants for vaccination. Currently, the gel of aluminum compound is almost the only adjuvant which is usable for vaccination for human. However, there are the following problems in regard to the aluminum adjuvant, and thus the adjuvants are in need of improvement. Specifically, since the quality of aluminum adjuvant tends to vary from lot to lot, large-scale production is unsuitable for this type of adjuvant. In addition to this, the compound is not easy to handle in the treatment with column and, as a result, the purification is not easy to be done. Another problem associated with its effect is that the compound is not effective for inducing the cellular immunity while it excels in inducing the humoral immunity. Thus there are limitations on the types of antigens to be used together with the adjuvant.

Studies and development of new types of adjuvants such as saponin are proceeding in order to overcome the drawbacks. Some illustrations are as follows.
1. Surface active substances such as saponins, etc.
2. Bacterial toxins such as cholera toxin, etc.
3. Constituents of microorganisms or plants such as BCG, muramyl peptide, etc.
4. Cytokines such as interleukins, etc.
5. Synthetic polyanion, polycation, etc.
6. Micro-carriers, etc.

Saponin is a generic name of compound group of triterpene glycosides and steroid glycosides that are contained in plants. It has been long known that some fractions containing saponin from plant have the adjuvant activity. However, saponins have complex structures and exhibit pleiotropic activities in addition to the adjuvant activity. Thus, it has been hard to identify which saponin compound has the adjuvant activity. The identification is not easy to be done even today.

For example, 20 types of saponin compounds have been isolated from Quil A, one of saponin fractions from a plant *Quillaja saponaria.* Of the compounds, some constituents including QS-21 have been revealed to exhibit the adjuvant activity (Published Japanese Translation of International Publication NO. Hei 2-504266). QS-21 strongly induces the cellular immunity and has been studied as an adjuvant capable of compensating for the defects of aluminum adjuvant. However, it is hard to purify QS-21; its structure is complicated; and also its solubility is too low. Thus, it is demanded to develop more effective, inexpensive, and safe adjuvant. For example, saponins generally exhibit strong hemolytic activity. The hemolysis causes side effects including anemia, organ malfunctions, malnutrition, thrombosis, and others. Therefore, particularly when administrated by injection, saponin can cause the problems.

Further, the present inventors have been found that some extracts of Chinese and Japanese traditional (Kampo) medicine consisting of several crude drugs exhibit the adjuvant activity and increase the titer of antibody against influenza virus in the nasal irrigation liquid and in the serum when used as an ingredient of influenza vaccine to be inoculated intranasally (H. Yamada and T. Nagai, Methods and Findings in Experimental and Clinical Pharmacology, 20(3), 185-192, 1998). However, it still remains to be clarified which compound has the adjuvant activity in the extracts.

### Disclosure of the Invention

An objective of the present invention is to provide a novel method for enhancing the immunological activity of vaccine in order to produce vaccines of which immunological activity is not reduced by lowering their dosage or by altering the vaccination route. More specifically, the objective is to screen an effective and safe saponin compound having a simpler structure among crude drug saponins and to thereby develop a novel adjuvant. Chinese and Japanese traditional (Kampo) medicine has long been used clinically in China, Japan, and other Asian countries, and its effectiveness and safety have been already established. Thus, the medicine is excellent and suitable as the material to be utilized for the present objective.

In other words, the objective of the present invention is to provide a group of saponins as novel, effective, and safe adjuvants,. to provide vaccines containing the saponins, and to contribute to the manufacture of effective and safe vaccines.

To attain the objective, the present inventors screened hot-water extracts of two hundred and tens of crude drugs for extracts exhibiting the adjuvant activity when used together with intranasally inoculated influenza vaccine. The screening revealed that hot-water extract of a crude drug "Polygalae Radix" exhibited the highest activity. Further, the inventors identified active constituents extracted and separated from the hot-water extract of Polygalae Radix by comparing their structures with those of known compounds utilizing NMR and high performance liquid chromatography. Consequently, they have found that compounds with a specific structure have a strong immunological stimulating activity, which would be highly safe, thereby completing the present invention. Specifically, the present invention relates to the following adjuvant and vaccine preparations containing the adjuvant:
(1) an adjuvant comprising a saponin compound having a presenegenin skeleton substituted with a substituted or unsubstituted sugar residue at position 28, where the substituted sugar residue essentially comprises an apiose residue as its substituent when the substituted sugar residue is tetra-substituted;
(2) the adjuvant of (1), wherein the substituted or unsubstituted sugar residue directly linked to the presenegenin skeleton at position 28 is a sugar residue containing 3 or more carbon atoms;
(3) the adjuvant of (2), wherein the sugar residue is a substituted or unsubstituted fucose residue;
(4) the adjuvant of (3), wherein the saponin compound is represented by the formula: 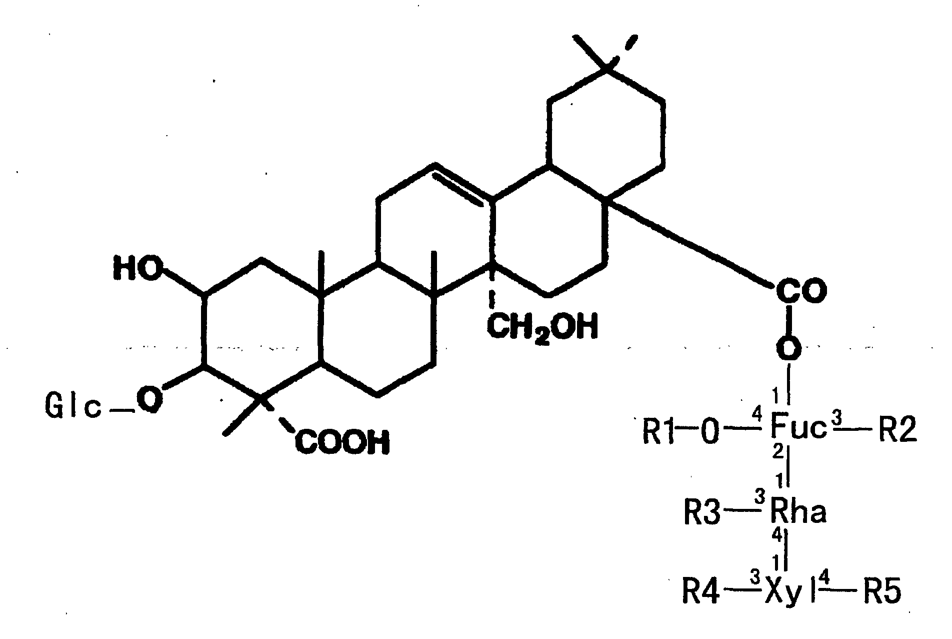 wherein Glc indicates glucose residue; Fuc, fucose residue; Rha, a rhamnose residue; Xyl, xylose residue; R1, monomethoxycinnamate residue or trimethoxycinnamate residue; R2, H or rhamnose residue; R3, H or apiose residue; R4, H or arabinose residue; and R5, H or galactose residue;
(5) the adjuvant of (4) , wherein the adjuvant comprises at least one of the saponin compounds selected from the group consisting of:
   (a) a compound where R1 is monomethoxycinnamate residue, R2 is rhamnose residue, R3 is apiose residue, R4 is H, and R5 is galactose residue;
   (b) a compound where R1 is trimethoxycinnamate residue, R2, R3, and R4 are H, and R5 is galactose residue;
   (c) a compound where R1 is trimethoxycinnamate residue, R2 is H, R3 is apiose residue, R4 is arabinose residue, and R5 is H; and
   (d) a compound where R1 is trimethoxycinnamate residue, R2 is H, R3 is apiose residue, and R4 and R5 are H;
(6) the adjuvant of any one of (1) to (5), wherein the saponin compound is prepared from a crude drug;
(7) a vaccine preparation comprising the adjuvant of any one of (1) to (6);
(8) the vaccine preparation of (7), wherein the vaccine preparation is to be inoculated intranasally or orally; and
(9) the vaccine preparation of (8), wherein the vaccine preparation comprises, as a vaccine, antigens from one or more pathogenic microorganisms selected from the group consisting of influenza virus, rotavirus, measles virus, rubella virus, mumps virus, AIDS virus, *Bordetella pertussis,* diphtheria bacillus, *Helicobacter pylori*, enterohaemorrhagic *Escherichia coli* (EHEC), *Chlamydia, Mycoplasma,* Malaria *Plasmodium*, coccidium, and schistosome.

The present invention further relates to use of a saponin compound having a presenegenin skeleton substituted with a substituted or unsubstituted sugar residue at position 28, where the substituted sugar residue essentially comprises an apiose residue as its substituent when the substituted sugar residue is tetra-substituted, as an adjuvant or for the production of an adjuvant. The present invention still further relates to use of the adjuvant in vaccine preparations or for the production of vaccine preparations.

Saponins used as the adjuvants of the present invention are a group of compounds having a presenegenin skeleton, which belong to saponins having oleanane skeleton. The skeleton is indicated as olean-12-ene-23,28-dioic acid,2,3,27-trihydroxy-(2β,3β,4α) in CAS nomenclature.

Examples of saponins having the adjuvant activity and of which structures have been clarified include QS-21, etc. However, the inventive saponin is unambiguously different from QS-21 and derivatives thereof. QS-21 belongs to another group of saponins with oleanane skeleton, but its skeleton is quillaic acid to which a variety of sugars and others are linked. The quillaic acid skeleton is indicated as olean-12-en-28-oic acid,3,16-dihydroxy,23-oxo-(3β,4α,16α) in CAS nomenclature. In other words, the presenegenin skeleton of saponins used as the inventive adjuvant is 23,28-dicarboxyl and has hydroxyl groups at positions 2, 3, and 27. On the other hand, the quillaic acid skeleton is 23-aldehyde-28-carboxyl and has hydroxyl groups at positions 3 and 16. The structures of the two are thus clearly different from each other.

Further, the above-mentioned saponin constituting the inventive adjuvant is a novel saponin adjuvant, because the sugar residue directly linked to the carboxyl group at position 28 of the presenegenin skeleton is substituted with a specific acyl group and because the sugar residue essentially comprises an apiose residue as its substituent when the sugar residue is substituted with 4 or more substituents. Herein, the number of substitutions for the above-mentioned sugar residue means the number of substituents including the linkage to the skeleton. The sugar residue linked to the presenegenin skeleton at position 28 is preferably a sugar with 3 or more carbon atoms, sugar alcohol, or sugar acid. Specifically, such sugar residues include glycerose residue, erythrose residue, fucose residue (referred to as Fuc), glucose residue (referred to as Glc), and sedoheptulose residue. Among them, when the sugar residue is fucose residue, the compounds include excellent saponins with low hemolytic activity. When the sugar linked to the carboxyl group at position 28 is fucose residue, the particularly preferred saponins are as shown in the above-mentioned formula. The sugar residue linked to the presenegenin skeleton at position 28 may further have an arbitrary substituent such as an acyl group or a sugar residue. Such acyl groups include monomethoxycinnamate residue (monomethoxycinnamoyl group; referred to as MC) and trimethoxycinnamate residue (trimethoxycinnamoyl group; referred to as TC) . Alternatively, it can be an organic acid such as lactic acid, pyruvic acid, succinic acid, or the like; or higher fatty acid such as oleic acid, etc. Further, a compound in which this acyl group *per* se is substituted with an amino group, hydroxyl group, nitro group, nitrile group, phosphate group, halogen, etc. can be illustrated. In addition, a compound in which the acyl group is linked to a sugar residue can be included. On the other hand, the sugar residue as a substituent includes rhamnose residue (referred to as Rha), apiose residue (referred to as Api), fucose residue, arabinose residue (referred to as Ara), xylose residue, glucose residue (referred to as Glc), glucuronic acid residue, galacturonic acid residue, mannose residue, mannuronic acid residue, and galactose residue. Each of these sugar residues can usually be linked, at one or more of positions 2, 3, and 4, to the above-mentioned fucose residue, which is linked to the presenegenin skeleton at position 28. For example, a compound in which R1 and R2 of the above-mentioned formula are substituted with an acyl group and a sugar residue, respectively, can be illustrated.

It is impossible to predict that saponins having the above-mentioned structure exhibit the strong adjuvant activity even based on descriptions in previous reports. For example, a study on the correlation between structure and activity for QS-21 has shown that the 23-aldehyde on the quillaic acid skeleton plays an important role in the onset of the activity (S. Soltysik, J.-Y. Wu, J. Recchia, D.A. Wheeler, M.J. Newman, R.T. Coughlin and C.R. Kensil, Vaccine, 13, 1403-1410, 1995). However, despite the fact that the saponins of the present invention have no aldehyde group but a carboxyl group at position 23, they have high adjuvant activities. This novel finding was eventually revealed based on the results of studies for long years by the present inventors.

The saponin constituting the inventive adjuvant is a compound having a presenegenin skeleton specified by the above-mentioned structure. Specific examples of the saponin that has a presenegenin skeleton and that is to be used in the present invention include onjisaponins A, E, F, and G. The structures and production methods of these compounds are already known (S. Sakuma and J. Shoji, Chemical and Pharmaceutical Bulletin, 29, 2431-2441, 1981; S. Sakuma and J. Shoji, Chemical and Pharmaceutical Bulletin, 30, 810-821, 1981). However, it has previously been unknown that these compounds have adjuvant activities for vaccination. For specifying the structures of these compounds, the substituents for R1 to R5 are summarized in Table 1. Abbreviations used in the table are the same as those of the substituents mentioned above. For example, regarding the onjisaponins indicated in the table, the number of substituents in the above-mentioned fucose residue, which is linked to the presenegenin skeleton at position 28, is as follows: A: 4, E: 3, F: 3, and G: 3.

**Table 1.**

| Structure of the onjisaponins of the present invention | | | | | |
|---|---|---|---|---|---|
| Onjisaponin | R1 | R2 | R3 | R4 | R5 |
| A | MC | Rha | Api | H | Gal |
| E | TC | H | H | H | Gal |
| F | TC | H | Api | Ara | H |
| G | TC | H | Api | H | H |

Saponins to be used in the present invention can be extracted, separated, purified, and produced from natural sources, for example, medicinal plants such as a crude drug by combining publicly known methods. Alternatively, they can be produced by chemical synthetic means. Procedures for the production are exemplified as follows.

*Polygalae Radix,* which is a saponin-containing crude drug, *Polygala tenuifolia* Willd., which is the original plant thereof, or any one of other plants belonging to the same genus is subjected to the extraction with a lower alcohol, such as methanol, ethanol, butanol, or the like, an organic solvent such as chloroform or the like, or water. The solvent is distilled off from the extract, and then the extract is purified by silica gel column chromatography or the like to give saponin to be used in the present invention. Alternatively, in some cases, the plant is subjected to the extraction with a water-containing organic solvent. The resulting extract is defatted with hydrocarbon such as hexane, and then the sample is partitioned with chloroform, butanol, or a mixed solvent such as water/chloroform or water/butanol. The organic solvent, in which soluble material extracted is contained, is distilled off, and then the extract is purified by silica gel column chromatography or the like to give the saponin. If desired, recrystallization with methanol or the like can be utilized for the purification.

There is no particular limitation on the form of usage of the inventive adjuvant as an active ingredient of vaccine. Specifically, it is possible to choose any of publicly known various adequate usage forms. The adjuvant can be physically mixed with or chemically linked to an antigen protein. Alternatively, the adjuvant may be incorporated in a carrier such as liposome. The inventive adjuvant may comprise a single saponin or multiple saponins.

Further, the inventive adjuvant can concurrently be used with one or more publicly known adjuvants. For instance, a pertussis-diphtheria-tetanus combined vaccine containing the inventive saponin and aluminum adjuvant is shown in Example 5. The inventive adjuvant can induce the mucosal immunity even when used with aluminum adjuvant. The type of saponin to be used as the inventive adjuvant or publicly known adjuvant to be preferably combined with the inventive one can empirically be selected by considering conditions such as the type of antigen used as an immunogen, the type of animal to be inoculated, the safety, or others. Thus, desired immunoreactivity can be enhanced as well as adverse side effects can be reduced by lowering amounts of both the antigen and the other adjuvant.

The hemolytic activity of saponin is one of indexes of the safety to be considered. Among the above-mentioned saponins, saponins with low hemolytic activity, for example, onjisaponins E, F, and G, are advantageous particularly when used to human. These saponins have a common structure in that the sugar residue linked to the carboxyl group at position 28 is fucose and further in that R2 is H in the fucose residue. However, the hemolytic activity of a saponin such as onjisaponin A is not acceptable. Nonetheless, it can be used, for example, when a vaccination route, by which the compound is slowly transferred into the blood, is selected, or the number of adjuvant inoculations is restricted within a range of acceptable level. Alternatively, the compound may be converted to a derivative with lower hemolytic activity (for example, onjisaponin E) by using a publicly known chemical modification method. The compound can be utilized as a material to give other derivatives.

New vaccine preparations are provided by utilizing the inventive adjuvant. The vaccine preparations of the present invention include vaccines in both narrow and broad senses. Specifically, the vaccines include: i) vaccines in a narrow sense, which are effective to infectious diseases of human and other animals caused by virus, bacterium, fungus, protozoan, other microorganisms. Such vaccines are exemplified by various vaccines such as influenza vaccine, pertussis vaccine, purified pertussis-diphtheria-tetanus combined vaccine, Japanese encephalitis vaccine, hepatitis B vaccine, rotavirus vaccine, measles vaccine, rubella vaccine, mumps vaccine, measles-rubella-mumps combined vaccine, measles-rubella combined vaccine, and *Haemophilus influenzae* vaccine. The vaccines also include multi-drug resistant Staphylococcus aureus (MRSA) vaccine, *Helicobacter pylori* (abbreviated as H. *pyroli* hereafter) vaccine, enterohaemorrhagic *Escherichiacoli* (EHEC) vaccine, *Salmonella* vaccine, *Chlamydia* vaccine, *Mycoplasma* vaccine, AIDS vaccine, malaria vaccine, coccidium vaccine, and schistosome vaccine. Further, ii) the vaccines in a broad sense are exemplified by vaccines, which are effective in the prevention and treatment of non-infectious diseases, such as cancer vaccine, infertility vaccine, gastric ulcer vaccine, diabetic vaccine, and arteriosclerotic vaccine.

These vaccines include various vaccines that are categorized based on the types of methods to produce them. Specifically, the vaccines include attenuated live vaccine, inactivated vaccine, component vaccine, vaccine using DNA, etc. The vaccine using DNA includes vaccines containing DNA fragment integrated in a carrier such as plasmid and vaccines used in combination with ribozyme or antisense oligonucleotide, and the like. The permeability of DNA fragment, ribozyme, or antisense oligonucleotide can be enhanced by the inventive saponin adjuvant. These vaccines can be used for prevention or treatment. The vaccines also include recombinant vaccines containing, as their active ingredient, the antigen produced in biological cells engineered by gene recombination techniques. These vaccines may be plain vaccines or combined vaccines. The production methods and usage forms of the vaccines are exemplified as follows.

Influenza vaccine; split vaccine containing hemagglutinin (HA), neuraminidase (NA), nuclear protein (NP), matrix protein (M), or a part of these that is obtained by the following steps: growing the viruses in embryonated eggs or in Vero cells by using animal cell culture techniques; degrading the viruses with ether, detergent, etc.; and purifying or that is obtained by gene recombination techniques or chemical synthesis; or DNA vaccine for intranasal inoculation that contains DNA fragments containing genes encoding these proteins.

Pertussis vaccine; inactivated vaccine that is obtained by the following steps: culturing *Bordetella pertussis*, treating the culture supernatant or bacteria by salting-out, ultracentrifugation, and others to extract constituents of interest, and detoxicating with formalin; or vaccine containing mutant pertussis toxin (PT), filamentous hemagglutinin (FHA), 69 K membrane protein, or a partial peptide of these that is prepared by gene recombination techniques or prepared as a product of artificial mutant strain obtained by the treatment with a mutagenizing agent.

Pertussis-diphtheria-tetanus combined vaccine; this is a triple vaccine prepared by mixing pertussis vaccine with diphtheria toxoid and tetanus toxoid.

Japanese encephalitis vaccine; inactivated vaccine that is obtained by the following steps: growing the viruses in the mouse brain or in Vero cells by using animal cell culture techniques; purifying the virus particles by ultracentrifugation or with ethyl alcohol, and inactivating the virus with formalin; or vaccine containing antigen proteins obtained by gene recombination techniques or chemical synthesis.

Hepatitis B vaccine; plasma vaccine that is obtained by separating and purifying HBs antigen by salting-out and ultracentrifugation using blood collected from hepatitis B carriers as raw material; or recombinant vaccine containing the antigen portions obtained by gene recombination techniques or chemical synthesis.

Measles vaccine; live vaccine of attenuated virus that is prepared by growing the virus in culture cells such as chicken fetal cells or in embryonated eggs; recombinant vaccine containing a part of the virus; or recombinant vaccine containing the protective antigen prepared by gene recombination techniques or chemical synthesis.

Rubella vaccine; vaccine containing the viruses grown in culture cells such as chicken fetal cells or in embryonated eggs, a part of the virus, or the protective antigen prepared by gene recombination techniques or chemical synthesis.

Mumps vaccine; attenuated live vaccine containing the viruses grown in culture cells such as rabbit cells or in embryonated eggs, a part of the virus, or the protective antigen prepared by gene recombination techniques or chemical synthesis.

Measles-rubella combined vaccine; dual vaccine that is obtained by mixing measles vaccine and rubella vaccine.

Measles-rubella-mumps combined vaccine; triple vaccine that is obtained by mixing measles vaccine, rubella vaccine, and mumps vaccine.

Rotavirus vaccine; vaccine containing the viruses grown in culture cells such as MA104 cell, the viruses collected from patient's feces, a part of the viruses, or the protective antigen prepared by gene recombination techniques or chemical synthesis.

*Mycoplasma* vaccine; vaccine containing *Mycoplasma* grown inmedium for *Mycoplasma,* a part thereof, or the protective antigen prepared by gene recombination techniques or chemical synthesis.

AIDS vaccine; vaccine containing the viruses grown in culture cells, the viruses obtained from patients, a part of these, or the protective antigen prepared by gene recombination techniques or chemical synthesis; or DNA vaccine containing effective DNA fragments.

*H. pylori* vaccine; vaccine containing, as antigens, lysate of cultured *H. pylori*, or urease, heat shock protein, toxin, and others separated from cultured *H. pylori;* or vaccine for injection, oral inoculation, or intranasal inoculation that comprises these antigen proteins produced by gene recombination techniques.

The above vaccines are provided as liquid forms or powdered forms. If desired to be powdered, the vaccines can be prepared as pharmaceutical preparations by a method including freeze-drying. Liquid forms of the pharmaceutical preparations are often suitable for the intranasal inoculation (intranasal spray, intranasal instillation, spread, etc.) and injection. Alternatively, the intranasal inoculation can be done by a method with powder spray. The inventive vaccine preparations can also be formulated with a publicly known stabilizer or preservative. The stabilizer includes about 0.1 to 0.2% gelatin or dextran, 0.5 to 1% sodium glutamate, about 5% lactose, about 2% sorbitol, etc. Known preservatives include about 0.01% thimerosal and about 0.1% β-propiolactone.

The mixing ratio between vaccine antigen and saponin can be, for example, 1:0.0001 to 1:10,000 (weight ratio) in vaccine preparations of the present invention. The range is just a typical example. Asuitable ratio is selected depending on the type of vaccine. Methods required for the selection are known to those skilled in the art.

The inventive vaccine preparation can be prepared by mixing the above-mentioned immunogen with the inventive adjuvant at an adequate mixing ratio. The preparation must be done under strictly sterile conditions. Each of raw materials must be completely sterile. As the matter of course, contaminated proteins that are unnecessary for vaccination and that act as pyrogens or allergens must be removed as much as possible. Methods to achieve the treatment are known to those skilled in the art. The inventive vaccine preparation can be effective even when the vaccine antigen and the inventive saponin are separately prepared as pharmaceutical preparations and then the two are mixed with each other at the time of inoculation or the two are separately inoculated at almost the same time.

### Method of vaccination

The vaccine preparation of the present invention can be utilized by any of publicly known methods.

The dose is preferably 5 to 50 µl in intranasal inoculation to mouse. The dose is preferably 0.1 to 1.0 ml in inoculation to human by intranasal administration or injection. The dose is changeable when desired. Regarding the combination with immunological antigen, for example, it has been believed that the following immunological antigens are advantageously inoculated intranasally or orally in terms of vaccination effect or inoculation procedure:
influenza virus, rotavirus, measels virus, rubella virus, mumps virus, human immunodeficiency virus, *Bordetella pertussis*, diphtheria bacillus, *H. pylori,* enterohaemorrhagic *Escherichia coli* (EHEC), *Chlamydia*, *Mycoplasma*, *Plasmodium,* coccidium, and schistosome.

These immunological antigens can be inoculated singly or concurrently, for example, like pertussis-diphtheria-tetanus triple vaccine or measels-rubella dual vaccine. The intranasal and oral inoculations are preferable, because mucous membranes of the respiratory tract and digestive tract can be infection routes. It is preferable to use a suitable adjuvant exhibiting the activity of enhancing the immunological actions in order to induce the immunity in local mucous membranes, which are infection routes. Further, some vaccinations, such as vaccination against *Plasmodium,* are performed in regions without sufficient medical facilities in most cases. In such occasions, it is advantageous to select vaccination routes such as intranasal or oral inoculation route, because a person who is not a medical technician such as physician or nurse can perform the vaccination.

### Brief Description of the Drawings

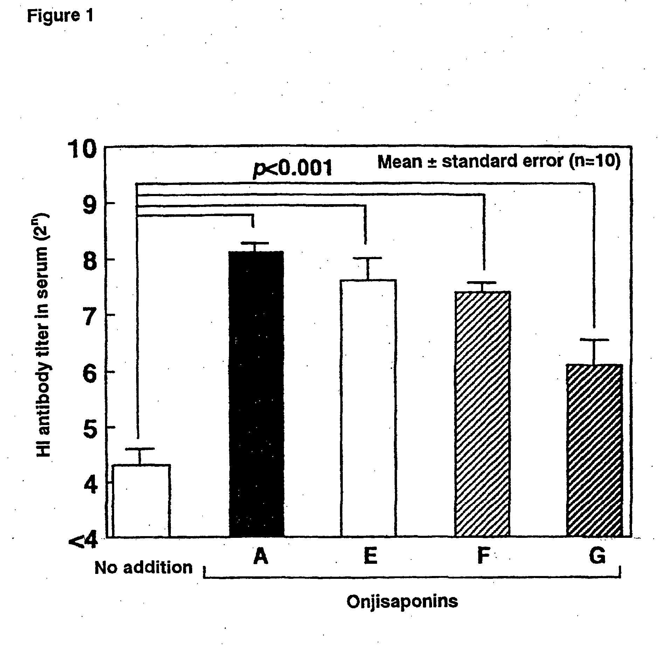

Figure 1 indicates a graph showing the results of primary production of antibody in the serum when an influenza vaccine was intranasally inoculated as the inventive vaccine preparations. In this figure, the ordinate indicates the antibody titer (2ⁿ HI unit) and the abscissa indicates the type of adjuvant used.

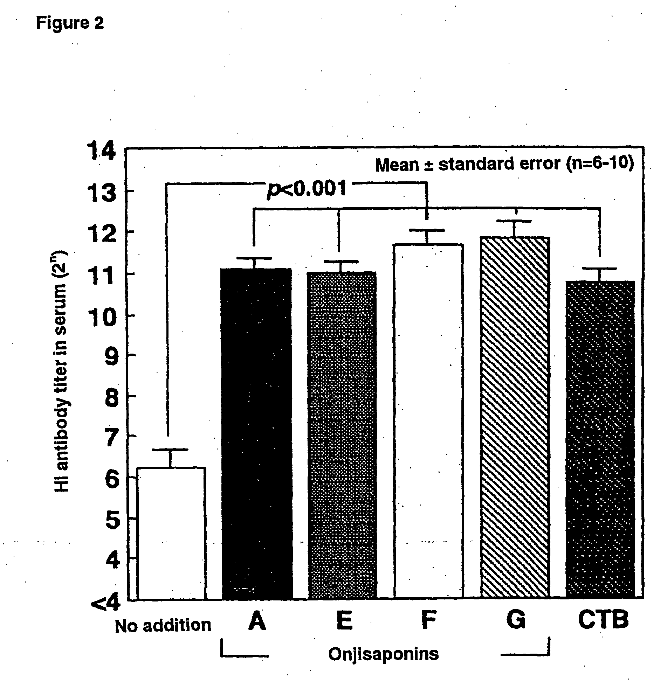

Figure 2 indicates a graph showing the results of secondary production of antibody in the serum when an influenza vaccine was intranasally inoculated as the inventive vaccine preparations. In this figure, the ordinate indicates the antibody titer (2ⁿ HI unit) and the abscissa indicates the type of adjuvant used.

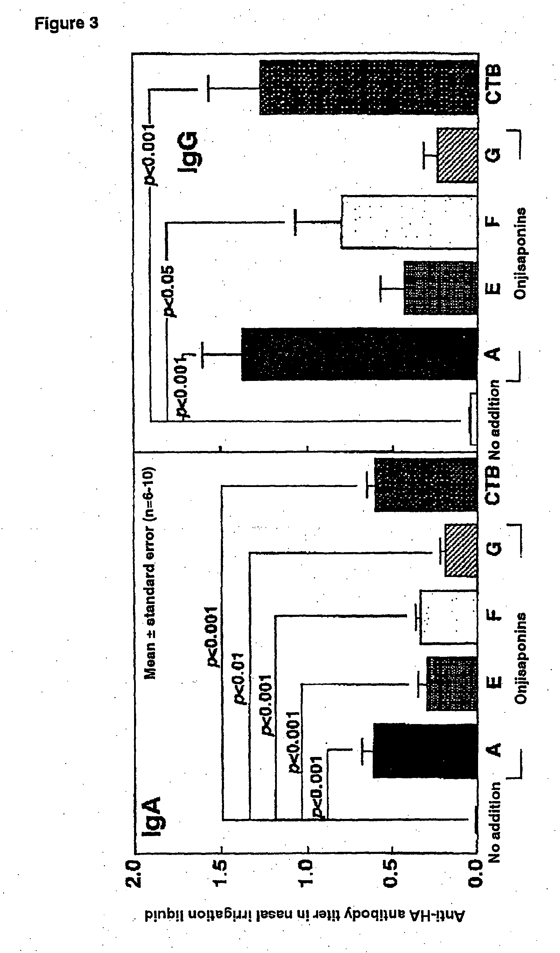

Figure 3 indicates a graph showing the results of secondary production of antibody in the nasal irrigation liquid when an influenza vaccine was intranasally inoculated as the inventive vaccine preparations. In this figure, the ordinate indicates the antibody titer (ELISA unit) and the abscissa indicates the type of adjuvant used.

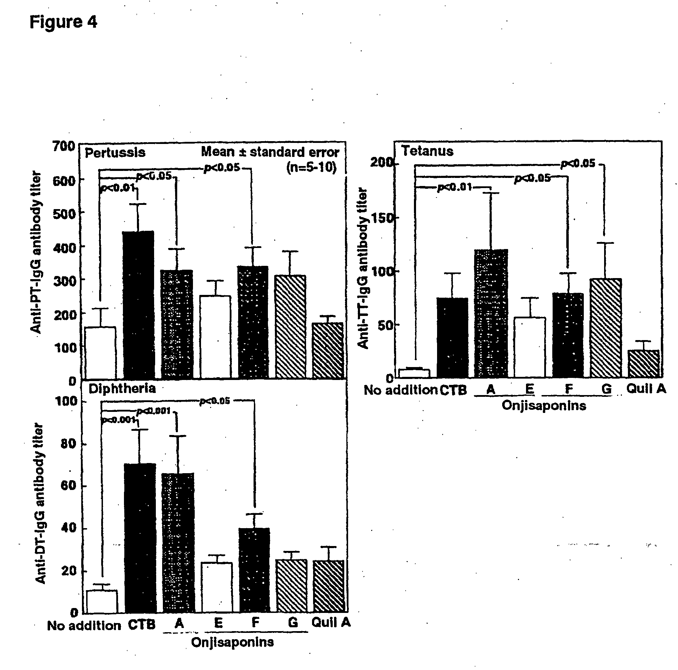

Figure 4 indicates a graph showing the results of secondary production of antibody in the serum when a pertussis-diphtheria-tetanus combined vaccine was intranasally inoculated as the inventive vaccine preparations. In this figure, the ordinate indicates the antibody titer (ELISA unit) and the abscissa indicates the type of adjuvant used.

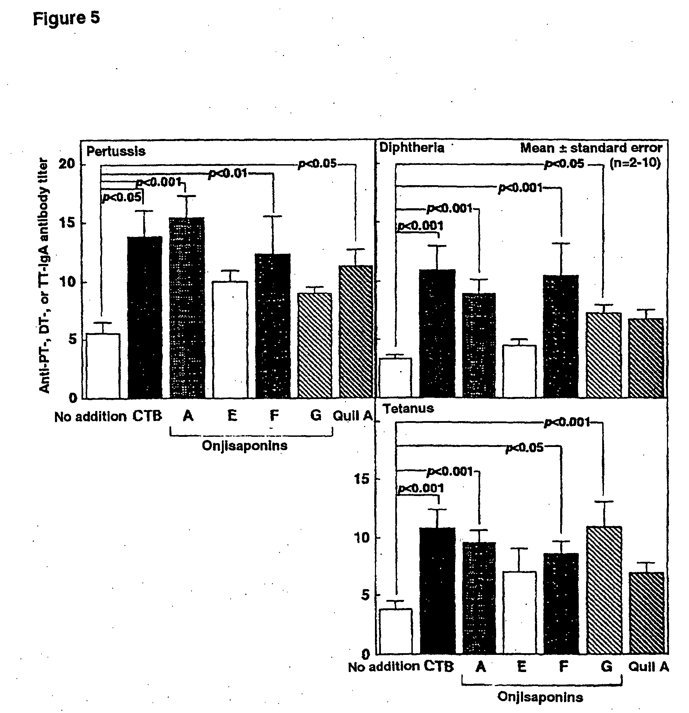

Figure 5 indicates a graph showing the results of secondary production of antibody in the nasal irrigation liquid when a pertussis-diphtheria-tetanus combined vaccine was intranasally inoculated as the inventive vaccine preparations. In this figure, the ordinate indicates the antibody titer (ELISA unit) and the abscissa indicates the type of adjuvant used.

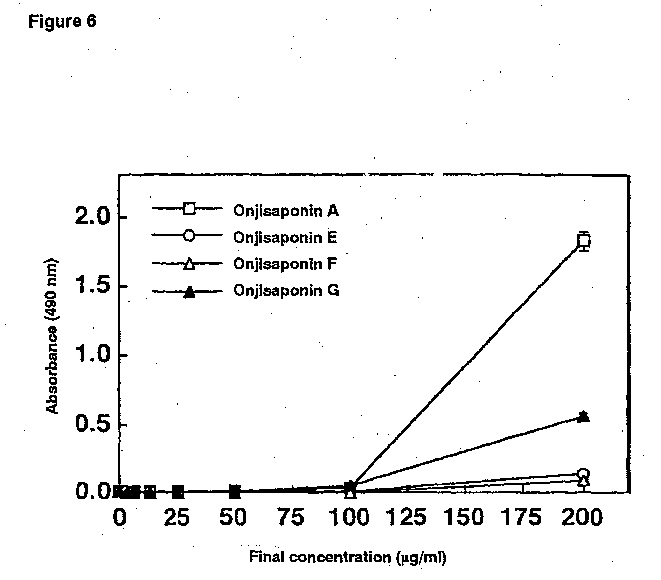

Figure 6 indicates a graph showing hemolytic activity of the inventive adjuvant. In this figure, the ordinate indicates the amount of released hemoglobin (absorbance at 490 nm) and the abscissa indicates the final concentration (µg/ml) of saponin.

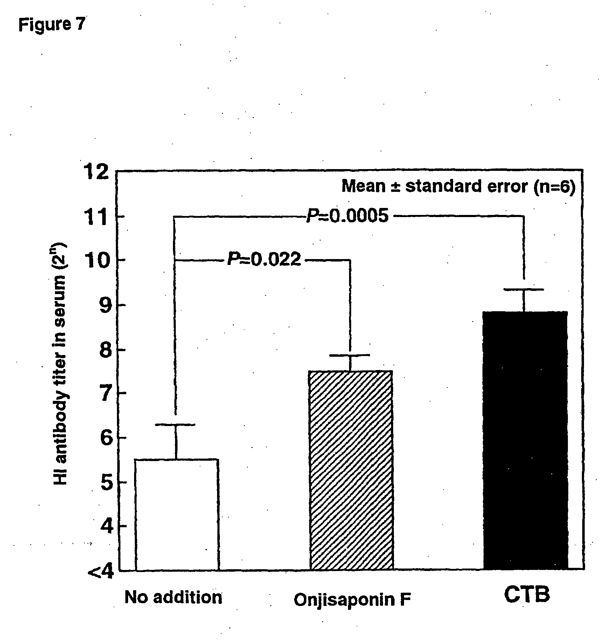

Figure 7 indicates a graph showing the results of secondary production of antibody in the serum when an influenza vaccine was intranasally inoculated as the inventive vaccine preparations together with the low-dosage adjuvant. In this figure, the ordinate indicates the antibody titer (2ⁿ HI unit) and the abscissa indicates the type of adjuvant used.

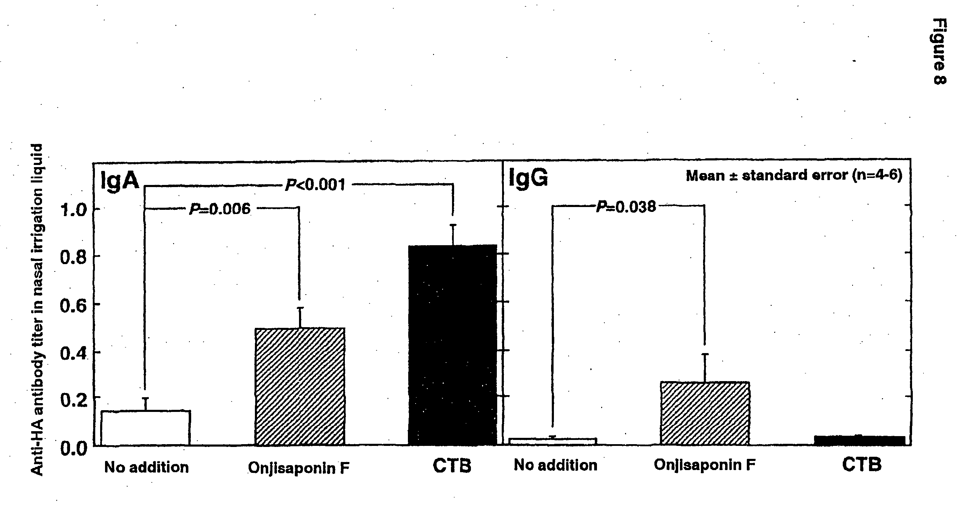

Figure 8 indicates a graph showing the results of secondary production of antibody in the nasal irrigation liquid when an influenza vaccine was intranasally inoculated as the inventive vaccine preparations together with the low-dosage adjuvant. In this figure, the ordinate indicates the antibody titer (ELISA unit) and the abscissa indicates the type of adjuvant used.

### Best Mode for Carrying out the Invention

The present invention is illustrated below with reference to Examples, but is not to be construed as being limited thereto.

### Example 1. Preparation of saponin (method 1)

A preparation method is described for onjisaponins A, E, F, and G. Onjisaponins A, E, F, and G were prepared according to the method of Shoji et al. (S. Sakuma and J. Shoji, Chemical and Pharmaceutical Bulletin, 29, 2431-2441, 1981; S. Sakuma and J. Shoji, Chemical and Pharmaceutical Bulletin, 30, 810-821, 1981).

Specifically, Polygalae Radix (500 g) was refluxed with 1 liter of methanol and then a methanol-soluble fraction was yielded. The residue was repeatedly treated 6 times in the same manner. The methanol-soluble fraction was then dried under reduced pressure to give a methanol extract (yield, 150 g; recovery rate, 30%). The extract was dissolved in distilled water, and then the resulting solution was subjected to shaking extraction with 1 liter of benzene 3 times to remove the lipid fraction. The resulting aqueous layer was further subjected to shaking extraction with 1.5 liters of water-saturated butanol 6 times to give a butanol-soluble fraction. The butanol-soluble fraction was dried under reduced pressure, and then the resulting residue was re-dissolved in 3 liters of butanol. The solution was concentrated to an about 1/5 volume under reduced pressure. The resultant precipitate was collected as a crude saponin fraction (yield, 40 g; recovery rate, 8%). The crude saponin fraction was further fractionated by silica gel column chromatography using water-saturated butanol to give a mixture fraction (Fr. 1) of onjisaponins D, E, F, and G and amixture fraction (Fr. 2) of onjisaponins A, B, and C. Each of Frs. 1 and 2 was fractionated by silica gel column chromatography [eluate, chloroform:methanol:ethanol:10% acetic acid = 8:4:1:2 (the upper layer was used)]. Fr. 1 yielded crude onjisaponin F fraction, G fraction, and a mixture fraction of D and E. Fr. 2 yielded crude onjisaponin A fraction, B fraction, and C fraction. The mixture fraction of onjisaponins D and E was further fractionated by silica gel column chromatography [eluate, butanol:ethylacetate:water=4:1:2 (the upper layer was used)] to give crude onjisaponin D fraction and E fraction. The crude onjisaponin fractions were separately further purified by using Sephadex LH-20 (eluate, methanol) to give purified onjisaponins A (recovery rate, 0.52%), B (recovery rate, 0.77%), C (recovery rate, 1.07%), D (recovery rate, 0.29%), E (recovery rate, 0.28%), F (recovery rate, 0.94%), and G (recovery rate, 0.11%).

### Example 2. Preparation of saponin (method 2)

Preparation method with a variety of high performance liquid chromatography (HPLC) was tested for the production of onjisaponins A, E, F, and G.

Specifically, 500g of Polygalae Radix were decoctedwith 10 liters of distilled water. After the water was reduced to half that volume, the mixture was filtered with stainless mesh. The resulting residue was again treated with 10 liters of distilled water in the same manner. The extracts were mixed together and filtered with a glass fiber filter. The resulting filtrate was subjected to freeze-drying treatment to give a hot-water extract. The hot-water extract (133.90 g) was dissolved in 1.79 liters of distilled water, and then subjected to centrifugation to give a supernatant. Four times as much volume of ethanol was added to the supernatant. Ethanol precipitation was carried out by stirring the mixture at room temperature overnight. The solution was allowed to stand still for a day to fractionate the precipitation. The precipitated material was separated from the supernatant. Further, the residual precipitate that remained to be separated was fractionated by centrifugation. The obtained supernatants were mixed together. The solvent was distilled off under reduced pressure to give a fraction originating from ethanol-precipitation supernatant. The fraction originating from ethanol-precipitation supernatant was re-dissolved in 1 liter of water. The solution was subjected to shaking extraction with 600 ml of hexane to remove lipid fraction. The resulting aqueous layer was further subjected to shaking extraction with 1 liter of chloroform and subsequently with 3 liters of water-saturated n-butanol. Then, the solvent was distilled off under reduced pressure to give a chloroform-soluble fraction or a butanol-soluble fraction.

Each of these chloroform-soluble and butanol-soluble fractions was suspended in water of 1 liter, and then dialyzed against distilled water for 10 days by using a dialysis membrane (exclusion molecular weight, 10,000). The inner dialysate solvent was distilled off under reduced pressure to give a chloroform-soluble or a butanol-soluble non-dialyzed active fraction. The resulting fractions were further fractionated by HPLC using a hydroxyapatite column. The fractionation was performed by using a mixed solvent of water and acetonitrile as an eluate to give a mixture of onjisaponins A, E, F, and G.

The active mixture fractions were further fractionated by HPLC using a phenyl column. The fractionation was carried out by using a mixed solvent of water and acetonitrile as an eluate to give fractions containing onjisaponins A, E, F, and G. By this purification method, onjisaponins A, E, F, and G can be obtained without the hydroxyapatite-HPLC procedure.

It is possible to verify the effectiveness of saponin with presenegenin skeleton as the adjuvant by using a publicly known biological method. The following Examples demonstrate that onjisaponins A, E, F, and G are effective as the adjuvant for enhancing the antibody production for a variety of vaccines.

### Example 3. Enhancing effect on the production of antibody against influenza HA vaccine inoculated intranasally

Purified influenza viruses (A/PR/8/34 strain) were treated with ether to remove lipid constituents. The resulting HA vaccine (the amount of HA was 0.5 mg/ml) was mixed with the same volume of saline solution (1 mg/ml) of onjisaponins A, E, F, or G purified by the method as shown in Example 1 to give a vaccine preparation. The purity of each fraction of onjisaponin used was at least about 95% or higher. BALB/c mice (7-week-old females) were anesthetized with amobarbital. The vaccine preparation (20 µl) was dropped into the left nasal cavity with a micro-pipette. After 4 weeks, whole blood was collected from the heart of each mouse under ether anesthesia to prepare the serum. The serum was first treated with RDE (receptor destroying enzyme) to remove nonspecific hemagglutinating substances. The serum was serially 2-fold diluted in U-shaped wells of a micro-titer plate. Each was mixed with 16 HA unit of the virus. After the mixed sample was allowed to stand still for 30 minutes at room temperature, chicken erythrocytes were added thereto. The mixture was allowed to stand still at room temperature for an hour. Then, the titer of hemagglutination inhibiting (HI) antibody was evaluated.

Figure 1 shows the. influence of onjisaponins A, E, F, and G on the HI antibody titer in the serum. When the HA vaccine alone was inoculated intranasally, the HI antibody was detected to be at a low level. However, when the HA vaccine was inoculated together with onjisaponins A, E, and F, the HI antibody titer was elevated in the serum 8 to 14 times relative to that in the case of the vaccine alone. In addition, onjisaponin G increased 3 to 5 times the HI antibody titer. These findings suggest that the onjisaponins can enhance the production of antibody against the coexisting influenza HA vaccine.

### Example 4. Enhancing effect on the secondary production of antibody against influenza HA vaccine

An influenza HA vaccine (1 mg/ml) prepared in the same manner as shown in Example 3 was mixed with the same volume of saline solution of the sample (1 mg/ml) to give a vaccine preparation. BALB/c mice (7-week-old females) were anesthetized by intraperitoneally inoculating sodium amobarbital. The vaccine preparation (20 µl) was intranasally inoculated to mice. After bred for 3 weeks, the mice were further subjected to secondary intranasal inoculation of the vaccine alone. After the mice were bred for further one week, sera and nasal irrigation liquids were prepared from them. The titer of anti-influenza virus antibody in the serum was evaluated based on the HI antibody titer. After bloodletting, the nasal irrigation liquids were collected from the mice by perfusing the right and left nasal cavities twice with 1 ml of phosphate buffered saline (PBS) containing 0.1% bovine serum albumin (BSA). The quantities of anti-HA-IgA and anti-HA-IgG in the nasal irrigation liquids were determined by enzyme linked immunosorbent assay (ELISA). Prior to the assay for anti-HA-IgA, each well of a 96-well EIA plate (Linbro) was treated with 100 µl of HA vaccine (5 µg/ml) suspended in a coating buffer (0.01 M carbonate-sodiumbicarbonate buffer, pH9.6). The plate was allowed to stand still for the coating at room temperature for 2 hours. The plate was then washed with Tween-20-containing PBS (abbreviated as PBS-Tween hereafter). Subsequently, each well was coated with 200 µl of PBS containing 1% BSA and 0.1% NaN₃. The plate was allowed to stand still at 4°C overnight, and then washed with PBS-Tween. Subsequently, a 100-µl aliquot of protein G-Sepharose 4FF (Pharmacia-Biotech Co.) unadsorbed fraction of the nasal irrigation liquid was added to each well. The plate was allowed to stand still at room temperature for 2 hours and then washed with PBS-Tween. Subsequently, 100 µl of alkaline phosphatase-labeled goat anti-mouse IgA (α-chain specific, 1:1000) diluted with PBS containing BSA and 0.1% NaN₃ was added to each well. The mixture was allowed to stand still at room temperature overnight. Then, the plate was washed with PBS-Tween. Sodium p-nitrophenyl phosphate (1 mg/ml; Sigma Co.) dissolved in 10% diethanolamine buffer (pH 9.8) was added to each well. The mixture was allowed to stand still at room temperature for 20 to 30 minutes. Then, the color development was monitored with O.D. at 405 nm in a plate reader (type MRX-MD, Dynex Co). The assay for anti-HA-IgG was carried out by using a protein G-Sepharose 4FF-adsorbed fraction of the nasal irrigation liquid as a sample and by using an alkaline phosphatase-labeled rabbit anti-IgG (γ-chain specific, 1:1000) as a secondary antibody.

Figure 2 shows the influence of onjisaponins A, E, F, and G on the production of anti-influenza virus antibody in the serum in secondary response. The onjisaponins A, E, F, and G increased the HI antibody titer in the serum 27 to 50 times as compared with the case where the HA vaccine alone was used. The adjuvant activity of the onjisaponins was comparable to that of the same amount of cholera toxin B subunit (CTB; Unexamined Published Japanese Patent Application NO. Hei 2-243633) used as a positive control.

Figure 3 shows the influence of onjisaponins A, E, F, and G on the production of anti-influenza virus IgA and IgG antibodies in the nasal irrigation liquid in secondary response. The anti-HA-IgA and IgG antibodies were detected at very low levels in the primary intranasal inoculation of the vaccine alone. On the other hand, the anti-HA-IgA and IgG antibody titers in the nasal irrigation liquid was increased to the highest level in a group primarily inoculated with the vaccine in combination with onjisaponin A, which was comparable to that found in a group primarily inoculated with the vaccine in combination with CTB. In addition, while onjisaponin F increased the production of both anti-HA-IgA and IgG antibodies, onjisaponins E and G increased only the production of anti-HA-IgA to a statistically significant level.

These findings suggest that the onjisaponins used in the primary inoculation have the activities of very strongly enhancing antibody production induced by the secondary inoculation. In other words, this means that the onjisaponins strongly induce the memory effect on the coexisting HA vaccine.

### Example 5. Enhancing effect on the production of antibody against pertussis-diphtheria-tetanus combined vaccine

The saline solution (1 mg/ml) of onjisaponin used in Example 3 was mixed with a pertussis-diphtheria-tetanus combined vaccine (containing 15 µg/ml of pertussis vaccine, 2 LF/ml of tetanus toxoid, 20 LF/ml of diphtheria toxoid, and 0.27 mg/ml of aluminum hydroxide gel; Kitasato Institute) to give a vaccine preparation. BALB/c mice (7-week-old females) were anesthetized by intraperitoneally inoculating sodium amobarbital. The vaccine preparation (20 µl) was intranasally inoculated to each mouse. The same amount of the vaccine was further inoculated to each mouse after 4 weeks. The mice were bred for 2 weeks and then the sera and nasal irrigation liquids were collected from the mice. The evaluation of antibody titer was performed by ELISA for anti-pertussis toxin (PT)-IgG, anti-diphtheria toxoid (DT)-IgG, and anti-tetanus toxoid (TT)-IgG antibodies in the sera and for anti-PT-IgA, anti-DT-IgA, and anti-TT-IgA antibodies in the nasal irrigation liquids.

Figure 4 shows the influence of onjisaponins A, E, F, and G on the antibody titers of anti-PT-IgG, anti-DT-IgG, and anti-TT-IgG in the serum. The respective titers of anti-PT-IgG, anti-DT-IgG, and anti-TT-IgG antibodies were found to be at low levels in a group subjected to the inoculation of pertussis-diphtheria-tetanus combined vaccine alone. On the other hand, the titers of anti-PT-IgG, anti-DT-IgG and anti-TT-IgG antibodies in the serum were increased to highest levels in a group subjected to the primary inoculation of a mixture of the vaccine and onjisaponin A, which were comparable to those in a group subjected to the inoculation of the vaccine and CTB (cholera toxin B subunit). While onjisaponin F also elevated the titers of anti-PT-IgG, anti-DT-IgG, and anti-TT-IgG antibodies in the serum, onjisaponin G increased only anti-TT-IgG antibody titer to a statistically significant level in the serum. When used in the same quantity as those of other samples, a saponin mixture, Quil A, derived from the tree bark of *Quillaja saponaria,* exhibited no adjuvant activity for the titers of anti-PT-IgG, anti-DT-IgG, and anti-TT-IgG antibodies in the serum.

Figure 5 shows the influence of onjisaponins A, E, F, and G on the titers of anti-PT-IgA, anti-DT-IgA and anti-TT-IgA antibodies in the nasal irrigation liquids. The antibody titers of anti-PT-IgA, anti-DT-IgA, and anti-TT-IgAwere found to be low in a group inoculated with a pertussis-diphtheria-tetanus combined vaccine alone. On the other hand, the titers of anti-PT-IgA, anti-DT-IgA, and anti-TT-IgA antibodies in the nasal irrigation liquid were increased to highest levels in a group primarily inoculated with the vaccine together with onjisaponin A or F, which were comparable to those in a group subjected to the inoculation of the vaccine and CTB. Onjisaponin G also elevated the titers of anti-DT-IgA and anti-TT-IgA antibodies in the nasal irrigation liquid. While Quil A slightly increased the titer of anti-PT-IgA antibody in the nasal irrigation liquid, it exhibited no adjuvant activity for the titers of anti-DT-IgA and anti-TT-IgA antibodies.

### Example 6. Hemolytic test for active saponins

It has been known that saponins generally have the hemolytic activity. Thus, the inventive active saponins were tested for the hemolytic activity as follows. The sheep erythrocytes were washed 3 times with phosphate buffered saline (PBS), and then diluted 2.5 times with the same buffer for the subsequent experiment. Aliquots (100 µl ) of solutions of onjisaponins A, E, F, and G (200, 100, 50, 25, 6.25, 3.125 µg/ml-PBS solution) were added to V-shaped wells of a 96-well micro-plate, and 25 µl of sheep erythrocyte suspension was added to each well. The plate was allowed to stand still at room temperature for 30 minutes. Then, the samples were centrifuged at 1000 rpm for 5 minutes. An aliquot (50 µl) of the resulting supernatant was transferred to a flat-bottomed well of a plate and the absorbance thereof was measured at 490 nm in a micro-plate reader (Model 450, BioRad Co.). The hemolytic activity of test compound was evaluated based on the increase in absorbance at 490 nm, which results from the release of hemoglobin from sheep erythrocytes. Figure 6 shows the hemolytic activities of onjisaponins A, E, F, and G. Large differences were found in the hemolytic activity among the compounds. The hemolysis was hardly recognized with onjisaponin E or F at a final concentration of 200 µg/ml. On the other hand, a moderate degree of hemolysis was observed with onjisaponin G and hemolysis clearly occurred with onjisaponin A.

### Example 7. Preparation of influenza HA vaccine-onjisaponin pharmaceutical preparation (injection)

Onjisaponins E and F were dissolved in PBS. The PBS solution was sterilized by filtration and then mixed with influenza HA vaccine (HA 1 mg/ml) so that 0.5 ml of the solution contained 5 to 10 µg of the influenza HA vaccine and 10 µg of the onjisaponins. The solution was added into containers, which was used as an influenza HA vaccine-onjisaponin injection. Such preparations can be stored at 10°C or lower in a cool and dark place.

The influenza HA vaccine and saponin prepared above were inoculated into mice. The antibody production was evaluated after 4 weeks. The test results showed that the titer of HI antibody was 2⁸ in the blood of mice subjected to the inoculation of influenza HA vaccine alone and that the titer was 2^{11.6} with saponin-containing vaccine.

### Example 8. Preparation of pertussis vaccine-onjisaponin pharmaceutical preparation (nasal drop)

Onj isaponin F was dissolved in PBS and the solution was sterilized by filtration. The solution was mixed with a pertussis vaccine so that 20 µl of the solution contained pertussis vaccine of which amount corresponded to 15 µg of protein nitrogen and 10 µg of the onjisaponin. A preservative (0.005% thimerosal) was added to the solution. The resulting mixture was added into containers, which was used as a pertussis vaccine-onjisaponin preparation to be used by intranasal inoculation. Such preparations should be stored at 10°C or lower in a cool and dark place.

The pertussis vaccine and saponin prepared above were intranasally inoculated into mice. The same amount of the vaccine was further inoculated to the mice after 4 weeks, and then the antibody production was evaluated. The test results showed that the titer of anti-PT-IgG antibodywas 156.3 ELISA units in the blood of mice subjected to the inoculation of pertussis vaccine alone and that the titer was 334.2 ELISA units with saponin-containing vaccine. While the titer of anti-PT-IgA antibody was 6 ELISA units in the nasal irrigation liquid of mice subjected to the inoculation of pertussis vaccine alone, the titer was 12 ELISA units with saponin-containing vaccine.

### Example 9. Preparation of hepatitis B vaccine-onjisaponin pharmaceutical preparation (injection)

Onjisaponins F and G were dissolved in PBS and sterilized by filtration. The solution was mixed with a hepatitis B vaccine so that 1 ml of the solution contained HBs antigen of which amount corresponded to 40 µg of protein and 10 µg of the onjisaponin. A preservative (0.01% thimerosal) and stabilizer (0.2% porcine gelatin) was added to the solution. The resulting mixture was added into containers, which was used as a hepatitis B vaccine-onjisaponin injection. Such preparations should be stored at 10°C or lower in a cool and dark place.

The hepatitis B vaccine and saponin prepared above were inoculated into mice. The antibody production in the blood was evaluated after 3 weeks. The test results by passive hemagglutination showed that the antibody titer was 2^{3.6} units in mice subjected to the inoculation of hepatitis B vaccine alone and was 2^{6.2} units with saponin-containing vaccine.

### Example10. Preparation of Japanese encephalitis vaccine-onjisaponin pharmaceutical preparation (injection)

Onjisaponins F and G were dissolved in PBS. The PBS solution was sterilized by filtration and then mixed with Japanese encephalitis vaccine so that 1 ml of the solution contained inactivated Japanese encephalitis virus particles corresponding to 10^{7.0} PFU and 10 µg of onjisaponins. A stabilizer (0.2% porcine gelatin) was added to the solution. The resulting mixture was added into containers, which was used as a Japanese encephalitis vaccine-onjisaponin injection. Such preparations should be stored at 10°C or lower in a cool and dark place.

The Japanese encephalitis vaccine and saponin prepared above were inoculated twice at a 1-week interval into mice. The antibody production in the blood was evaluated. The test results showed that the titer of neutralizing antibody was 10^{1.88} units in mice subjected to the inoculation of the Japanese encephalitis vaccine alone, and the titer was 10^{2.90} units with saponin-containing vaccine.

### Example 11. Preparation of measles vaccine-onjisaponin pharmaceutical preparation (nasal drop)

Onjisaponin E was dissolved in PBS and the solution was sterilized by filtration. The solution was mixed with a measles vaccine so that 20 µl of the solution contained measles vaccine of which amount corresponded to 20 µg of the virus particles and 2.5 µg of the onjisaponin. A stabilizer (0.2% porcine gelatin, 0.1% sodium glutamate, 5% lactose) was added to the solution. The resulting mixture was added into containers, which was used as a measles vaccine-onjisaponin nasal drop. Such preparations should be stored at 10°C or lower in a cool and dark place.

The measles vaccine and saponin prepared above were inoculated twice at a 3-week interval into mice. Then, the antibody production in the blood was evaluated. The test results showed that the ELISA antibody titer was 0.18 in mice subjected to the inoculation of measles vaccine alone, and the titer was 0.25 with saponin-containing vaccine.

### Example 12. Preparation of rubella vaccine-onjisaponin pharmaceutical preparation (nasal drop)

Onjisaponin E was dissolved in PBS and the solution was sterilized by filtration. The solution was mixed with a rubella vaccine so that 20 µl of the mixture contained rubella vaccine of which amount corresponded to 20 µg of the virus particles, and 2.5 µg of onjisaponin. A stabilizer (0.1% sodium glutamate, 5%lactose) was added to the solution. The resulting mixture was added into containers, which was used as a rubella vaccine-onjisaponin nasal drop. Such preparations should be stored at 10°C or lower in a cool and dark place.

The rubella vaccine and saponin prepared above were inoculated twice at a 3-week interval into mice. Then, the antibody production in the blood was evaluated. The test results showed that the ELISA antibody titer was 0.13 in mice subjected to the inoculation of the vaccine alone, and the titer was 0.850 with saponin-containing vaccine.

### Example 13. Preparation of mumps vaccine-onjisaponin pharmaceutical preparation (nasal drop)

Onjisaponin E was dissolved in PBS and the solution was sterilized by filtration. The solution was mixed with a mumps vaccine so that 20 µl of the solution contained mumps vaccine of which amount corresponded to 20 µg of the virus particles and 2.5 µg of the onjisaponin. Astabilizer (0.2% porcine gelatin, 0.1% sodium glutamate, 5% lactose) was added to the solution. The resulting mixture was added into containers, which was used as a mumps vaccine-onjisaponin nasal drop. Such preparations should be stored at 10°C or lower in a cool and dark place.

The mumps vaccine and saponin prepared above were inoculated twice at a 3-week interval into mice. Then, the antibody production in the blood was evaluated. The test results showed that the ELISA antibody titer was 0.023 in mice subjected to the inoculation of vaccine alone, and the titer was 0.045 with saponin-containing vaccine.

### Example 14. Preparation of measles-rubella combined vaccine-onjisaponin pharmaceutical preparation (nasal drop)

Onjisaponin E was dissolved in PBS and the solution was sterilized by filtration. The solution was mixed with measles-rubella vaccine so that 20 µl of the solution contained the respective vaccines of which amounts corresponded to 7 µg of the virus particles and 2.5 µg of the onjisaponin. A stabilizer (0.2% porcine gelatin, 0.1% sodium glutamate, 5% lactose) was added to the solution. The resulting mixture was added into containers, which was used as a measles-rubella vaccine-onjisaponin nasal drop. Such preparations should be stored at 10°C in a cool and dark place.

The measles-rubella vaccine and saponin prepared above were inoculated twice at a 3-week interval into mice. Then, the antibody production in the blood was evaluated. The test results showed that the ELISA antibody titer was 0.14 for measles or 0.090 for rubella in mice subjected to the inoculation of the vaccine alone, and the titer was 0.30 for measels or 0.29 for rubella with saponin-containing vaccine.

### Example 15. Preparation of rotavirus vaccine-onjisaponin pharmaceutical preparation (oral preparation, nasal drop)

Onjisaponins E and F were dissolved in PBS and the solution was sterilized by filtration. The solution was mixed with a rotavirus vaccine so that 20 µl of the solution contained rotavirus vaccine of which amount corresponded to 3.3 µg of the virus particles and 10 µg of the onjisaponin. The solution was added into containers, which was used as a rotavirus vaccine-onjisaponin oral preparation or nasal drop. Such preparations should be stored at 10°C or lower in a cool and dark place.

The rotavirus vaccine and saponin prepared above were inoculated twice at a 3-week interval into mice. Then, the antibody production in the blood was evaluated. The test results showed that the ELISA antibody titer was 0.089 with intranasal inoculation of the vaccine alone and the titer was 0.38 with saponin-containing vaccine and that the titer was 0.018 in the case of oral inoculation of the vaccine alone and it was 0.27 with saponin-containing vaccine.

### Example 16. Preparation of Mycoplasma vaccine-onjisaponin pharmaceutical preparation (injection)

Onjisaponins E and F were dissolved in PBS and the solution was sterilized by filtration. The solution was mixed with a *Mycoplasma* vaccine so that 1 ml of the solution contained *Mycoplasma* vaccine of which amount corresponded to 2.0x10¹⁰ CFU (colony forming unit) virus particles and 10 µg of the onjisaponins. The solution was added into containers, which was used as a *Mycoplasma* vaccine-onjisaponin injection. Such preparations should be stored at 10°C or lower in a cool and dark place.

The *Mycoplasma* vaccine and saponin prepared above were inoculated 3 times at 2-week intervals into mice. Then, observation was carried out for the lesions associated with *Mycoplasma* infection. The test results showed that the lesion was recognized in all the 10 mice subjected to the inoculation of the vaccine alone and that the lesion was found in 3 of the 10 mice subjected to the inoculation of the saponin-containing vaccine. The mean value of the number of lesions was 277 with the vaccine alone, and it was 142 with saponin-containing vaccine.

### Example 17. Enhancing effect of low-dosage onjisaponin on the secondary production of antibody against influenza HA vaccine

An influenza HA vaccine (0.1 mg/ml) prepared from purified influenza viruses (A/Beijing/262/95 strain) in the same manner as shown in Example 3 was mixed with the same volume of saline solution of the sample (0.1 mg/ml) to give a vaccine preparation. BALB/c mice (7-week-old females) were anesthetized by intraperitoneally inoculating sodium amobarbital. The vaccine preparation (20 µl) was intranasally inoculated to mice. After bred for 3 weeks, the mice were further subjected to secondary intranasal inoculation of a mixture of the vaccine and the sample. After the mice were bred for further one week, sera and nasal irrigation liquids were prepared from them. The titer of anti-influenza virus antibody in the serum was evaluated based on the HI antibody titer. After bloodletting, the nasal irrigation liquids were collected from the mice by perfusing the right and left nasal cavities twice with 1 ml of PBS containing 0.1% BSA. The quantities of anti-HA-IgA and anti-HA-IgG in the nasal irrigation liquids were determined by ELISA.

Figure 7 shows the influence of onjisaponin F on the production of anti-influenza virus antibody in the serum in secondary response. The onjisaponin F significantly increased HI antibody titer in the serum at a dose of 1 µg per mouse as compared with the case where the HA vaccine alone was used. The adjuvant activity of onjisaponin F was slightly weaker than that of the same amount of CTB used as a positive control.

Figure 8 shows the influence of onjisaponin F on the production of anti-influenza virus IgA and IgG antibodies in the nasal irrigation liquid in secondary response. The anti-HA-IgA and IgG antibodies were detected to be at low levels in the intranasal inoculation of the vaccine alone. On the other hand, onjisaponin F (1 µg/mouse) significantly increased the anti-HA-IgA and IgG antibody titers in the nasal irrigation liquid in a group inoculated with the vaccine in combination with onjisaponin F. The adjuvant activity of enhancing the titer of anti-HA-IgA antibody was lower for onjisaponin F than for the same amount of CTB. However, onjisaponin F significantly exhibited the adjuvant activity of enhancing the titer of anti-HA-IgG, while CTB exhibited no adjuvant activity.

These results suggest that, even at a low dosage, onjisaponin F used as an adjuvant strongly induces the antibody production in the serum and nasal cavity.

### Industrial Applicability

The Examples shown above clearly indicate the following.
1. The inventive adjuvant comprising onjisaponins can enhance the production of antibody against the coexisting influenza HA vaccine and pertussis-diphtheria-tetanus combined vaccine.
2. When inoculated together with the inventive adjuvant by intranasal route, vaccine antigen enhances not only the antibody production in the blood but also local antibody production (in the nasal irrigation liquid). In other words, the inventive adjuvant can reduce the inoculum dose of vaccine antigen to reduce the side effects.
3. The inventive adjuvant comprising onjisaponins exhibits, in the blood and in a local region (in the nasal irrigation liquid), higher activity of enhancing the antibody production than a saponin mixture, Quil A, derived from the tree bark of *Quillaja saponaria*, indicating that the saponin is more powerful as the adjuvant than Quil A.
4. The inventive adjuvant contains ingredients that exhibit substantially no hemolytic activity like, for example, onjisaponins E and F. In general, saponins exhibit strong hemolytic activity. However, some saponin compounds have no hemolytic activity as revealed in Example of the present invention. The present invention has great advantages revealing the fact that saponins having no hemolytic activity are separable from other saponins and further have the adjuvant activity.

As described above, vaccine preparation containing the inventive adjuvant is expected to be a highly safe agent that is effective for the prevention and treatment of virus infection and bacterial infection.

## Claims

1. An adjuvant comprising a saponin compound having a presenegenin skeleton substituted with a substituted or unsubstituted sugar residue at position 28, where the substituted sugar residue essentially comprises an apiose residue as its substituent when the substituted sugar residue is tetra-substituted.

2. The adjuvant of claim 1, wherein the substituted or unsubstituted sugar residue directly linked to the presenegenin skeleton at position 28 is a sugar residue containing 3 or more carbon atoms.

3. The adjuvant of claim 2, wherein the sugar residue is a substituted or unsubstituted fucose residue.

4. The adjuvant of claim 3, wherein the saponin compound is represented by the formula: 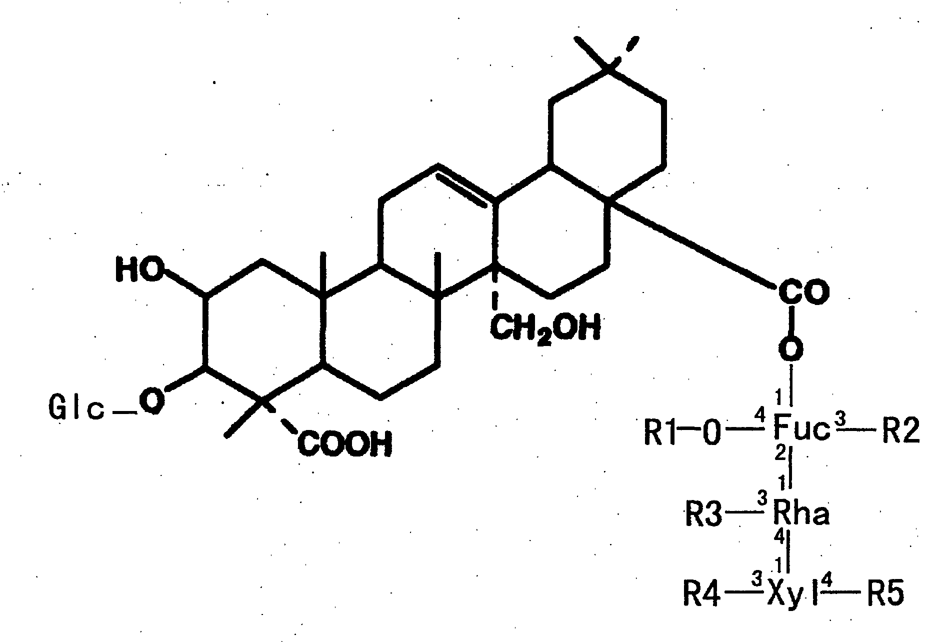 wherein Glc indicates glucose residue; Fuc, fucose residue; Rha, rhamnose residue; Xyl, xylose residue; R1, monomethoxy cinnamate residue or trimethoxycinnamate residue; R2, H or rhamnose residue; R3, H or apiose residue; R4, H or arabinose residue; and R5, H or galactose residue.

5. The adjuvant of claim 4, wherein the adjuvant comprises at least one of the saponin compounds selected from the group consisting of:
(a) a compound where R1 is monomethoxycinnamate residue, R2 is rhamnose residue, R3 is apiose residue, R4 is H, and R5 is galactose residue;
(b) a compound where R1 is trimethoxycinnamate residue, R2, R3, and R4 are H, and R5 is galactose residue;
(c) a compound where R1 is trimethoxycinnamate residue, R2 is H, R3 is apiose residue, R4 is arabinose residue, and R5 is H; and
(d) a compound where R1 is trimethoxycinnamate residue, R2 is H, R3 is apiose residue, and R4 and R5 are H.

6. The adjuvant of any one of claims 1 to 5, wherein the saponin compound is prepared from a crude drug.

7. A vaccine preparation comprising the adjuvant of any one of claims 1 to 6.

8. The vaccine preparation of claim 7, wherein the vaccine preparation is to be inoculated intranasally or orally.

9. The vaccine preparation of claim 8, wherein the vaccine preparation comprises, as a vaccine, antigens from one or more pathogenic microorganisms selected from the group consisting of influenza virus, rotavirus, measles virus, rubella virus, mumps virus, AIDS virus, *Bordetella pertussis,* diphtheria bacillus, *Helicobacter pylori,* enterohaemorrhagic *Escherichia coli* (EHEC), *Chlamydia, Mycoplasma*, *Plasmodium,* coccidium, and schistosome.
